Europäisches Patentamt
European Patent Office
Office européen des brevets

(19)

(11) Numéro de publication: 0 370 852 A1

(12) **DEMANDE DE BREVET EUROPEEN**

(21) Numéro de dépôt: **89403024.6**

(22) Date de dépôt: **03.11.89**

(51) Int. Cl.[5]: **C07D 471/04, A61K 31/435, //(C07D471/04,235:00,221:00)**

Revendications pour les Etats contractants suivants: ES + GR.

(30) Priorité: **08.11.88 FR 8814538**

(43) Date de publication de la demande: **30.05.90 Bulletin 90/22**

(84) Etats contractants désignés: **AT BE CH DE ES FR GB GR IT LI LU NL SE**

(71) Demandeur: **SYNTHELABO**
**58 rue de la Glacière**
**F-75013 Paris(FR)**

(72) Inventeur: **Manoury, Philippe**
**L'Orée de Verrières 38, Av. des Vaupépins**
**F-91370 Verrières Le Buisson(FR)**
Inventeur: **Binet, Jean**
**13, rue du Faubourg Saint-Nicolas**
**F-21121 Fontaine Les Dijon(FR)**
Inventeur: **Defosse, Gérard**
**29, rue de Tolbiac**
**F-75013 Paris(FR)**

(74) Mandataire: **Thouret-Lemaitre, Elisabeth et al**
**SYNTHELABO Service Brevets 58, rue de la Glacière**
**F-75013 Paris(FR)**

(54) **Dérivés de phényloxypropanolamines, leur préparation et leur application en thérapeutique.**

(57) Dérivés de phényloxypropanolamines, sous a forme de racémates ou d'isomères optiquement actifs, répondant à la formule (I)

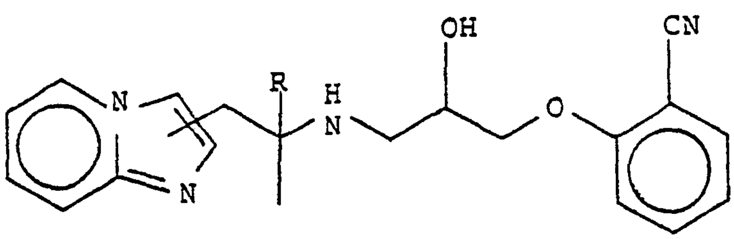

(I)

dans laquelle R = H ou $CH_3$,
ainsi que leurs sels d'addition aux acides pharmaceutiquement acceptables.
Application en thérapeutique.

EP 0 370 852 A1

# DERIVES DE PHENYLOXYPROPANOLAMINES, LEUR PREPARATION ET LEUR APPLICATION EN THERAPEUTIQUE

La présente invention a pour objet des dérivés de phénoxypropanolamines, leur préparation et leur application en thérapeutique.

Les composés de l'invention répondent à la formule (I)

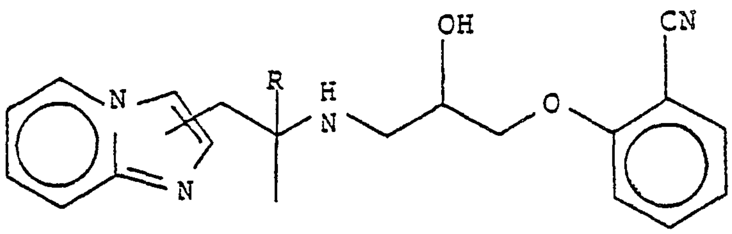

(I)

dans laquelle R est H ou $CH_3$,
et peuvent exister sous la forme de racémates ou d'isomères optiquement actifs.

Les sels d'addition des composés de l'invention aux acides pharmaceutiquement acceptables font partie de l'invention.

Selon l'invention on peut préparer les composés (I) selon le schéma réactionnel donné en annexe.
On fait réagir le composé (II) avec le composé (III), dans un solvant tel que l'éthanol, à la température de reflux.

Les exemples suivants illustrent l'invention.

Les analyses et les spectres IR et RMN confirment la structure des composés.

<u>Exemple 1</u>. Maléate de [[(imidazo[1,2-a]pyridinyl-3)-2 méthyl-1 éthyl]amino]-3 (cyano-2 phénoxy)-1 propanol-2.

1.1. (Nitro-2 propényl-1)-3 imidazo[1,2-a]pyridine.

On porte au reflux 30 mn, 10 g d'imidazo[1,2-a]pyridine-3 carboxaldéhyde avec 3,6 g d'acétate d'ammonium, 8 ml d'acide acétique et 15 ml de nitroéthane. On refroidit le mélange, filtre, lave avec de l'eau et sèche. F = 225° C (recristallisé du nitrométhane).

1.2. (Nitro-2 propyl-1)-3 imidazo[1,2-a]pyridine.

A 9 g du dérivé précédent en suspension dans 250 ml d'un mélange dioxanne-éthanol 50/50, on ajoute par portions 21 g de $NaBH_4$. On agite 1/2 h, évapore la solution. On reprend le résidu avec de l'eau, ajoute 3,5 ml d'acide acétique, extrait avec du chlorure de méthylène. On lave avec de l'eau, sèche, filtre, évapore. On purifie le produit par chromatographie. F = 88° C.

1.3. α-méthyl imidazo[1,2-a]pyridine-3-éthanamine.

On hydrogène dans un appareil de Parr, 6,7 g (0,033 mole) du dérivé nitré précédent en solution dans 125 ml d'éthanol en présence de 5 g de nickel de Raney. On filtre le catalyseur, évapore à sec. On prépare le chlorhydrate dans l'éthanol F > 300° C.

1.4. [[(Imidazo[1,2-a]pyridinyl-3]-2 méthyl-1 éthyl]amino]-3 (cyano-2 phénoxy)-1 propanol-2.

A une suspension de 4,3 g (0,0173 mole) du produit précédent dans 13 ml d'éthanol, on ajoute 6,9 ml de méthylate de sodium 5,05N ; on agite 5 mn et ajoute 3,4 g (0,02 mole) de (cyano-2 phénoxy)-1 époxypropane-2,3 puis porte au reflux 30 mn. On évapore à sec, reprend avec du chlorure de méthylène, lave avec de l'eau, sèche, filtre, évapore. On purifie le produit par chromatographie et prépare le fumarate dans l'acétone. F = 126° C.

Exemple 2. Maléate de [[[imidazo[1,2-a]pyridinyl-2] méthyl-1 éthyl]amino]-3 (cyano-2 phénoxy)-1 propanol-2.

2.1. [[Imidazo[1,2-a]pyridinyl-2]-2 méthyl-1 éthyl]-2 1H-isoindoledione-1,3.

A une solution de 15,3 g (0,163 mole) d'amino-2 pyridine dans 80 ml d'HMPT on ajoute 25,3 g (0,08 mole) de [(bromo-4 oxo-3 méthyl-1) propyl]-2 1H-isoindoledione-1,3. On agite la solution pendant 5 h, verse dans de l'eau, extrait avec de l'éther, lave la phase éthérée avec de l'eau, sèche, filtre, évapore.
On prépare le chlorhydrate dans l'éthanol chlorhydrique. F = 147° C.

2.2. α-méthyl imidazo[1,2-a]pyridine-2 éthaneamine.

On porte au reflux pendant 3 h, 24 g (0,07 mole) du composé précédent dans 240 ml d'HCl 6N. On évapore le mélange à sec puis on dissout le résidu dans de l'hydroxyde sodium 5N. La solution résultante est extraite avec du chlorure de méthylène. La solution d'extraction est séchée, filtrée et évaporée.
Eb = 165° C sous 0,04 mm de mercure.

2.3. [[(Imidazo[1,2-a]pyridinyl-2)-2 méthyl-1 éthyl]amino]-3 (cyano-2 phénoxy)-1 propanol-2.

On porte au reflux 1 h environ, 1,75 g (0,01 mole) de l'amine précédente sous forme de base et 1,8 g (0,01 mole) de (cyano-2 phénoxy)-1 époxypropane-2,3 dans 10 ml d'éthanol. On évapore le solvant, purifie l'huile résiduelle par chromatographie. On prépare le maléate dans l'éthanol.
F = 172° C (recristallisé du méthanol).

Exemple 3. Fumarate de [[(imidazo[1,2-a]pyridinyl-3)-2 diméthyl-1,1 éthyl]amino]-3 (cyano-2 phénoxy)-1 propanol-2.

3.1. (Nitro-2 méthyl-2 propyl-1)-3 imidazo[1,2-a]pyridine.

On porte au reflux 16 h, 144 g (0,44 mole) d'iodure de [imidazo[1,2-a]pyridinyl]-3 triméthyl méthaneammonium (J.G. Lombardino J. Org. Chem. 30, 2403-7, 1965) 48,5 ml (0,53 mole) de nitro-2 propane et 90 ml de méthylate de sodium 5,04 N.
On évapore le mélange à sec, on met en suspension le résidu obtenu dans de l'eau, puis, filtre le précipité.
On reprend le filtrat avec 3 volumes d'eau ; une gomme colorée précipite et cristallise.
Après purification par chromatographie sur colonne de silice, on obtient le produit F = 131° C.

3.2. α,α-diméthyl[imidazo[1,2-a]pyridine-3-éthanamine.

En utilisant la même méthode que celle décrite sous 1.3. à partir de 12,7 g (0,058 mole) du dérivé précédent on obtient le produit sous forme de chlorhydrate. F > 300° C.

3.3. Fumarate de [[imidazo[1,2-a]pyridinyl-3)-2 diméthyl-1,1 éthyl]amino]-3 (cyano-2 phénoxy)-1 propanol-2.

On porte au reflux 6,9 g (0,0365 mole) de produit précédent avec 7,6 g (0,043 mole) de (cyano-2

phénoxy)-1 époxypropane-2,3 dans 40 ml d'éthanol. On évapore le solvant et chromatographie le produit. On prépare le fumarate dans l'éthanol. F = 206 °C.

## Tableau

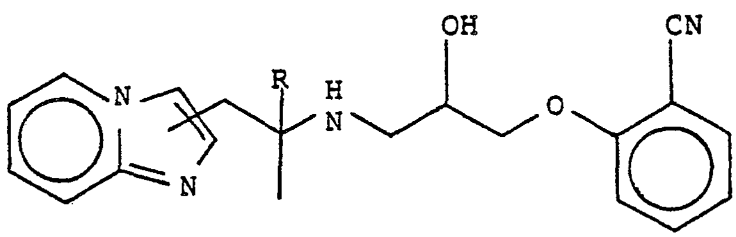

(I)

| Composé | R | liaison | sel | F(°C) |
|---|---|---|---|---|
| 1 | H | en 3 | fumarate | 126 |
| 2 | H | en 2 | maléate | 172 |
| 3 | $CH_3$ | en 3 | fumarate | 206 |

Les composés (I) de l'invention ont été soumis à une série d'essais pharmacologiques qui ont mis en évidence leurs propriétés intéressantes dans le domaine cardiovasculaire.

Les composés de l'invention ont été testés particulièrement pour leur activité antiglaucome.

La pression intraoculaire (PIO) aigue d'un jour a été évaluée sur des yeux normotendus et hypertendus de singes selon le test suivant : la pression intraoculaire (PIO) est déterminée à l'aide d'un Pneumatonograph Alcon après légère anesthésie de la cornée avec de la proparacaïne.

Après chaque mesure de pression intraoculaire, l'anesthésiant résiduel est éliminé par lavage avec une solution saline. Après mesure de la PIO de référence, le composé à tester ou le solvant est administré en deux apports de 25 microlitres aux deux yeux de 6 singes Cynomolgus.

Les mesures de PIO subséquentes sont faites 1, 3 et 7 heures après l'administration. Les yeux droits de tous les singes ont été traités par trabeculoplastie au laser plusieurs mois avant l'expérience et une hypertension oculaire s'est développée dans les yeux traités au laser.

Les animaux sont entraînés à s'asseoir dans des chaises entravées et sont conditionnés pour accepter les mesures de pression.

Les résultats sont donnés dans le tableau suivant :

Tableau 2

| Pression intraoculaire chez des singes Cynomolgus | | | | | |
|---|---|---|---|---|---|
| | | | Modification en % par rapport à la mesure de référence | | |
| Composé | Dose/oeil | oeil | 1 h | 3 h | 7 h |
| | | | après administration | | |
| ex. 1 | 500 µg | OD | -18,7 | -19,7 | -10,5 |
| | | OS | - 2,7 | - 3,1 | - 6,8 |
| ex. 3* | 500 µg | OD | -26,7 | -23,1 | -15,1 |
| | | OS | - 4,2 | - 0,7 | - 1,8 |
| ex. 3* | 500 µg | OD | -23,6 | -28,5 | - 5,4 |
| | | OS | - 5,0 | - 7,6 | - 3,1 |
| Remarques : | | | | | |
| OD = oeil laserisé<br>OS = oeil normal<br>N = 6<br>Tous les composés sont administrés sous la forme de solutions ou de suspensions dans un excipient. | | | | | |

* L'étude a été répétée sur des animaux différents.

Les composés de l'invention peuvent être utilisés pour le traitement du glaucome. Ils peuvent être présentés sous toute forme appropriée, en solutions ou en suspensions dans un excipient.

Annexe

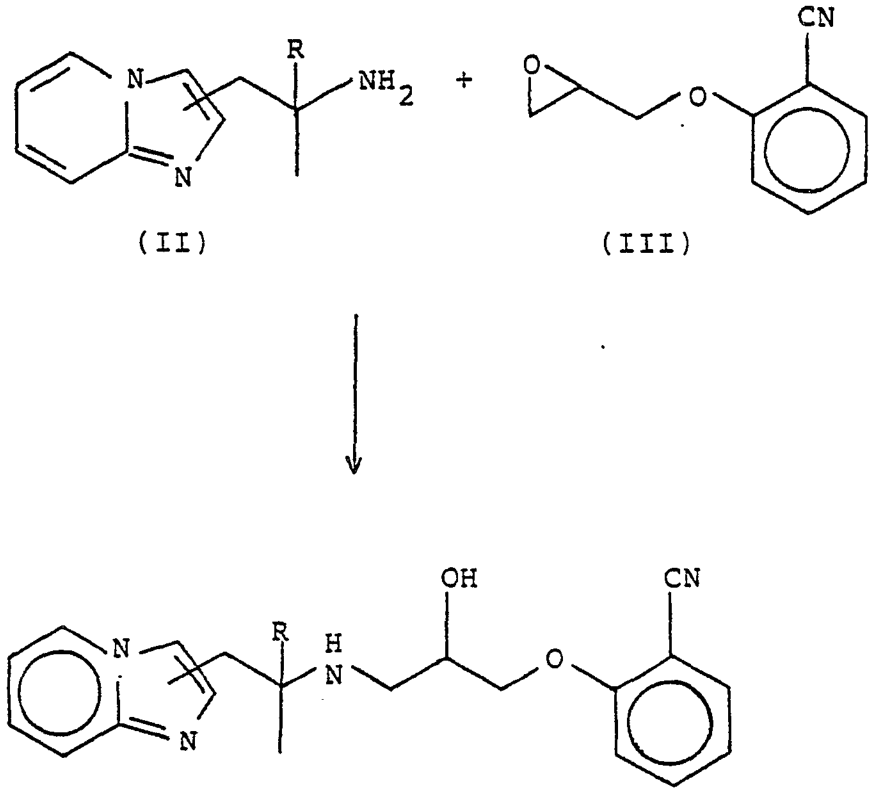

(II) (III)

(I)

**Revendications**

1. Dérivés de phényloxypropanolamines, sous la forme de racémates ou d'isomères optiquement actifs, répondant à la formule (I)

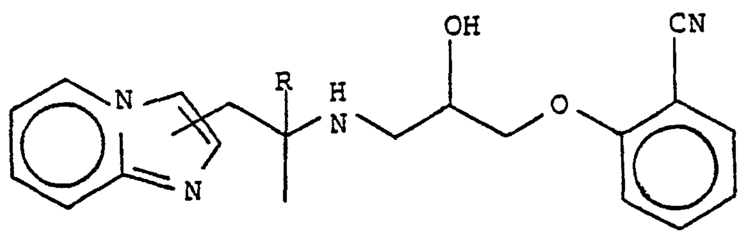

(I)

dans laquelle R est H ou $CH_3$,
ainsi que leurs sels d'addition aux acides pharmaceutiquement acceptables.

2. Procédé de préparation des composés selon la revendication 1, procédé caractérisé en ce que l'on fait réagir le composé (II)

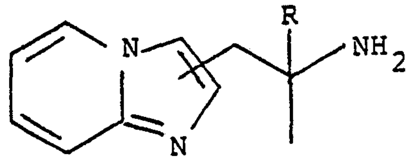

(II)

avec le composé (III)

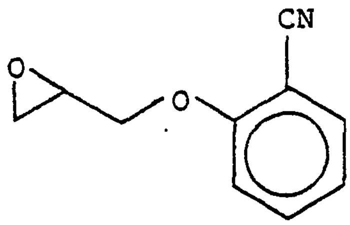

(III)

dans un solvant tel que l'éthanol, à la température de reflux.

3. Médicament caractérisé en ce qu'il contient un composé tel que spécifié dans la revendication 1.

4. Composition pharmaceutique caractérisée en ce qu'elle contient un composé tel que spécifié dans la revendication 1 en association avec tout excipient approprié.

Revendication pour les Etats contractants suivants: ES, GR.

Procédé de préparation de dérivés de phényloxypropanolamines, sous la forme de racémates ou d'isomères optiquement actifs, répondant à la formule (I)

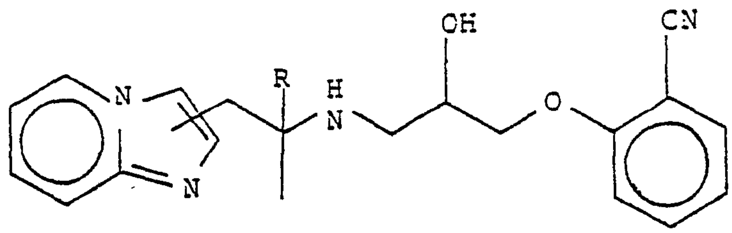

(I)

dans laquelle R est H ou $CH_3$,
procédé caractérisé en ce que l'on fait réagir un composé (II)

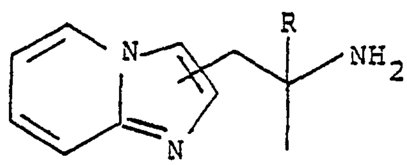

(II)

avec le composé (III)

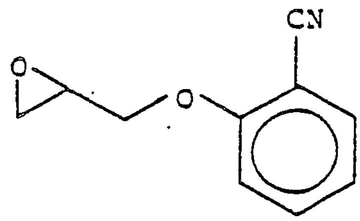

(III)

dans un solvant tel que l'éthanol, à la température de reflux.

Office européen des brevets

# RAPPORT DE RECHERCHE EUROPEENNE

Numero de la demande

EP 89 40 3024

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 006 614 (MERCK)<br>* Revendications 1,8 * | 1,4 | C 07 D 471/04<br>A 61 K 31/435//<br>(C 07 D 471/04<br>C 07 D 235:00<br>C 07 D 221:00 ) |
| Y | US-A-4 234 595 (MEAD JOHNSON)<br>* Revendications 1,98-100 * | 1,4 | |
| Y | FR-A-2 463 765 (CM INDUSTRIES)<br>* Revendications 1,4; page 13, comosé 7824 * | 1,4 | |

DOMAINES TECHNIQUES RECHERCHES (Int. Cl.5)

C 07 D 471/00
A 61 K 31/00

Le présent rapport a été établi pour toutes les revendications

| Lieu de la recherche | Date d'achèvement de la recherche | Examinateur |
|---|---|---|
| LA HAYE | 05-02-1990 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)